(19) Europäisches Patentamt
European Patent Office
Office européen des brevets

(11) **EP 4 183 327 B1**

(12) **FASCICULE DE BREVET EUROPEEN**

(45) Date de publication et mention
de la délivrance du brevet:
**17.07.2024   Bulletin 2024/29**

(21) Numéro de dépôt: **22202889.6**

(22) Date de dépôt: **21.10.2022**

(51) Classification Internationale des Brevets (IPC):
**A61B 5/00** *(2006.01)*      **A61B 5/16** *(2006.01)*
**G16H 50/00** *(2018.01)*      **A61M 21/00** *(2006.01)*

(52) Classification Coopérative des Brevets (CPC):
**A61B 5/4011; A61B 5/165; A61M 21/00;**
**G16H 40/63; G16H 50/70;** A61M 15/08;
A61M 21/0094; A61M 2021/0016; A61M 2021/005;
A61M 2205/3306; A61M 2205/3317;
A61M 2205/3368; A61M 2230/04; A61M 2230/06;
A61M 2230/40;                    (Cont.)

(54) **PROCÉDÉ DE CARACTÉRISATION D'UNE STIMULATION OLFACTIVE**

VERFAHREN ZUR CHARAKTERISIERUNG EINER GERUCHSSTIMULATION

METHOD FOR CHARACTERIZING OLFACTORY STIMULATION

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **17.11.2021   FR 2112153**

(43) Date de publication de la demande:
**24.05.2023   Bulletin 2023/21**

(73) Titulaires:
• **Robertet S.A.**
**06130 Grasse (FR)**
• **Université Côte d'Azur**
**06100 Nice (FR)**
• **Centre national de la recherche scientifique**
**75016 Paris (FR)**

(72) Inventeurs:
• **MELEQI, Xhino**
**06400 Cannes (FR)**

• **PEGARD, Anthony**
**06130 Grasse (FR)**
• **TOPIN, Jérémie**
**06000 Nice (FR)**

(74) Mandataire: **Germain Maureau**
**12, rue Boileau**
**69006 Lyon (FR)**

(56) Documents cités:
**WO-A1-2019/220428      US-A1- 2007 066 916
US-A1- 2008 255 949      US-A1- 2014 303 450**

(52) Classification Coopérative des Brevets (CPC): (Cont.)
A61M 2230/42; A61M 2230/50; A61M 2230/65

## Description

## Domaine de l'invention

La présente invention se rapporte à un procédé de caractérisation d'une stimulation olfactive, et plus particulièrement à la caractérisation d'une stimulation olfactive à l'aide d'un thème identifié.

## Art antérieur

De manière connue, le document US20120220857A1 concerne un procédé permettant de détecter la libération de dopamine suite à une stimulation olfactive. Il est ainsi possible de caractériser des fragrances en fonction de la libération, ou non de dopamine.

WO2019220428A1 concerne un système de surveillance et de modification d'un état émotionnel d'un sujet, comprenant au moins un capteur pour détecter des données, indicatrices d'une pluralité de caractéristiques biométriques d'un sujet qui sont associées à un état émotionnel actuel du sujet. Au moins un dispositif de stimulation est conçu pour générer un stimulus pour modifier l'état émotionnel actuel. Un processeur est conçu pour recevoir les données détectées, pour utiliser les données détectées pour calculer au moins un paramètre qui indique l'état émotionnel actuel du sujet, pour identifier une valeur dudit au moins un paramètre qui est indicateur d'un état émotionnel cible prédéterminé et, lorsque l'état émotionnel actuel indiqué est différent de l'état émotionnel cible, pour faire fonctionner le dispositif de stimulation pour générer un stimulus qui a été précédemment identifié comme facilitant le changement de l'état émotionnel actuel en l'état émotionnel cible.

Toutefois, ces solutions ne donnent pas une entière satisfaction.

En effet, la caractérisation présentée dans le document US20120220857A1 permet de caractériser les fragrances uniquement en fonction de la libération de dopamine, ce qui ne représente donc que deux catégories, les fragrances qui libèrent de la dopamine et celles qui ne le font pas.

La présente invention a pour but de résoudre tout ou partie des inconvénients mentionnés ci-dessus.

## Exposé de l'invention

A cet effet, la présente invention concerne un procédé de caractérisation d'une stimulation olfactive, le procédé comprenant :

- une pluralité de premières étapes de stimulation visuelle, chaque première étape de stimulation visuelle consistant à réaliser une stimulation visuelle, chaque stimulation visuelle étant associée à au moins un thème identifié ;
- une pluralité de premières étapes d'enregistrement de valeurs de variables physiologiques, chaque première étape d'enregistrement étant réalisée en continu pendant une première étape de stimulation visuelle parmi la pluralité de premières de stimulation visuelle, de façon à obtenir un premier enregistrement physiologique, la pluralité d'étapes d'enregistrement de valeurs de variables physiologiques résultant ainsi en l'obtention d'une pluralité de premiers enregistrements physiologiques, chaque premier enregistrement physiologique étant associé à un thème identifié correspondant au thème identifié associé à la première étape de stimulation visuelle ;

- une deuxième étape de stimulation olfactive, la deuxième étape de stimulation olfactive consistant à réaliser une stimulation olfactive ;
- une deuxième étape d'enregistrement de valeurs de variables physiologiques, la deuxième étape d'enregistrement de valeurs de variables physiologiques étant réalisée en continu pendant et postérieurement à la deuxième étape de stimulation olfactive résultant ainsi en l'obtention d'un deuxième enregistrement physiologique ;
- une étape de comparaison, l'étape de comparaison consistant à comparer les premiers enregistrements physiologiques avec le second enregistrement physiologique, de façon à isoler au moins un premier enregistrement physiologique, ledit au moins un premier enregistrement physiologique isolé étant proche du deuxième enregistrement physiologique ; et
- une étape de détermination, l'étape de détermination consistant à déterminer au moins un thème identifié, le thème identifié étant le thème identifié associé à l'au moins une première étape de stimulation visuelle isolé lors de l'étape de comparaison.

Au sens de la présente invention, une pluralité de premières étapes de stimulation visuelle, chaque première étape de stimulation visuelle consistant à réaliser une stimulation visuelle, chaque stimulation visuelle étant associée à au moins un thème identifié est une pluralité de premières étapes de stimulation, chaque première étape de stimulation consistant à réaliser une stimulation visuelle, chaque stimulation visuelle étant associée à au moins un thème identifié.

Au sens de la présente invention, une deuxième étape de stimulation olfactive, la deuxième étape de stimulation olfactive consistant à réaliser une stimulation olfactive est une deuxième étape de stimulation, la deuxième étape de stimulation consistant à réaliser une stimulation olfactive.

Au sens de la présente invention, un thème identifié peut-être de toutes sortes tel qu'un type d'émotion ou encore un genre cinématographique par exemple.

Selon un mode de réalisation, l'étape de stimulation visuelle est une étape dans laquelle une vidéo est montrée à un utilisateur. Une telle disposition permet une meilleure stimulation visuelle.

Selon un mode de réalisation, la vidéo est une vidéo en réalité virtuelle. Une telle disposition permet une meilleure immersion et donc une meilleure stimulation visuelle.

Selon un mode de réalisation, chaque stimulation visuelle a une durée de trente secondes. Une telle disposition permet de réaliser les étapes d'enregistrement de valeurs de variables physiologiques avec une meilleure précision. Cela permet par exemple de réaliser le calcul de la variabilité de la fréquence cardiaque sur des hautes fréquences ainsi que sur les basses fréquences. Au sens de la présente invention, les basses fréquences sont comprises entre 0,04 Hz et 0,15Hz inclus. Au sens de la présente invention, les hautes fréquences sont supérieures à 0,15Hz et inférieures à 0,4Hz inclus.

Selon un mode de réalisation, le thème identifié est un thème identifié par au moins un utilisateur. En d'autres termes, c'est un ensemble d'utilisateurs comprenant au moins un utilisateur qui choisit quel est le thème identifié associé à chaque stimulation visuelle. Et est donc propre à chaque utilisateur. Une telle disposition permet une bonne caractérisation d'une stimulation olfactive. Selon un mode de réalisation préféré, le thème identifié est identifié par au moins 50% des utilisateurs pour être considéré comme étant valide. Selon un mode de réalisation, lors de la deuxième étape de stimulation olfactive, le sens de la vue est restreint. Une telle disposition permet de limiter les perturbations telles que des stimulations visuelles naturelles et ainsi obtenir une meilleure stimulation olfactive.

Selon un mode de réalisation, la deuxième étape de stimulation olfactive consiste à approcher un élément qui produit une sensation sur l'odorat à sensiblement quatre centimètres du nez de l'utilisateur. Une telle disposition permet une bonne stimulation olfactive. Au sens de la présente invention, sensiblement quatre centimètres signifie entre trois centimètres et demi et quatre centimètres et demi.

Selon un mode de réalisation préféré, la deuxième étape de stimulation olfactive consiste à approcher un élément qui produit une sensation sur l'odorat au niveau d'une lèvre supérieure d'un utilisateur.

Selon un mode de réalisation, l'élément est contenu dans un flacon ou sur une mouillette. Au sens de la présente invention, une mouillette peut être de toutes sortes, afin que ladite mouillette puisse être imbibée d'un liquide, tel qu'un papier ou une éponge par exemple.

La deuxième étape d'enregistrement étant réalisée en continu pendant et postérieurement à la deuxième étape de stimulation olfactive de façon à obtenir un deuxième enregistrement physiologique, cela permet de comparer une première partie du deuxième enregistrement physiologique enregistrée pendant la deuxième étape de stimulation olfactive avec une deuxième partie du deuxième enregistrement physiologique afin de caractériser avec plus de précision ladite stimulation olfactive.

Selon un mode de réalisation, l'étape de détermination est réalisée à l'aide de toute méthode telle que d'une classification hiérarchique ascendante et/ou à l'aide à l'aide d'une analyse en composante principale et/ou à l'aide de la méthode des nuées dynamiques par exemple.

Les variables physiologiques peuvent être de toutes sortes telles que :

- la fréquence cardiaque dans les hautes et les basses fréquences afin de déterminer l'équilibre entre le système nerveux sympathique et parasympathique ;
- la réponse cutanée galvanique, afin de révéler un état d'éveil ou un état de stress par exemple ; et
- le rythme respiratoire afin de révéler un état physiologique et de conforter les états déduits par les autres variables physiologiques ou dérivés.

Selon un mode de réalisation, le procédé comprend une première étape d'enregistrement de mesures de référence, la première étape d'enregistrement de mesures de référence étant réalisée avant l'une quelconque des étapes de stimulation et consistant à mesurer en continu des valeurs de variables physiologiques afin d'obtenir un premier enregistrement de référence.

Selon un mode de réalisation, le procédé comprend une deuxième étape d'enregistrement de mesures de référence, la deuxième étape d'enregistrement de mesures de référence étant réalisée après ou pendant l'une quelconque des étapes de stimulation et consistant à mesurer en continu des valeurs de variables physiologiques afin d'obtenir un deuxième enregistrement de référence.

Selon un mode de réalisation, les enregistrements physiologiques comprennent des enregistrements réalisés à l'aide d'un photopléthysmogramme placé une phalange distale d'un majeur, et préférablement sur une phalange distale d'un majeur d'une main non dominante d'un utilisateur. Une telle disposition permet d'obtenir une mesure précise. Un photopléthysmogramme placé sur la phalange distale du majeur de la main non dominante permet de faciliter toute manipulation que l'utilisateur aurait à effectuer. En effet, ledit utilisateur serait alors apte à utiliser une main dominante.

Selon un mode de réalisation, les enregistrements physiologiques comprennent des enregistrements réalisés à l'aide d'un photopléthysmogramme placé une phalange distale d'un pouce, et préférablement de la main non dominante de l'utilisateur. Une telle disposition permet d'obtenir une mesure précise lorsque la phalange distale du majeur est trop petite. Un photopléthysmogramme placé sur la phalange distale du pouce de la main non dominante permet de faciliter toute manipulation que l'utilisateur aurait à effectuer. En effet, ledit utilisateur serait alors apte à utiliser une main dominante.

Selon un mode de réalisation, les enregistrements physiologiques comprennent des enregistrements réalisés à l'aide de deux électrodes de conductance électrodermale sur des phalanges médiales d'un index et d'un annulaire, et préférablement de la main non dominante de l'utilisateur. Une telle disposition permet d'obtenir une mesure précise. Placer les des électrodes de conductance électrodermale sur les phalanges médiales de l'index et de l'annulaire permet de faciliter toute manipulation que l'utilisateur aurait à effectuer. En effet, ledit utilisateur serait alors apte à utiliser une main dominante

Selon un mode de réalisation, les enregistrements physiologiques comprennent des enregistrements réalisés à l'aide d'un capteur de température cutanée sur une phalange distale d'un auriculaire, et préférablement de la main non dominante de l'utilisateur. Une telle disposition permet d'obtenir une mesure précise. Un capteur de température placé sur la phalange distale de majeur de la main non dominante permet de faciliter toute manipulation que l'utilisateur aurait à effectuer. En effet, ledit utilisateur serait alors apte à utiliser une main dominante.

Selon un mode de réalisation, un calcul de la moyenne de la température cutanée pour chacune des deux parties de chaque enregistrement physiologique est effectué.

Selon un mode de réalisation, les enregistrements physiologiques comprennent des enregistrements des volumes respiratoires.

Une telle disposition permet de déduire le rythme respiratoire.

Selon un mode de réalisation, les enregistrements des volumes respiratoires comprennent le rythme respiratoire ainsi que les volumes d'inspiration et d'expiration.

Une telle disposition permet de déduire l'état physiologique de l'utilisateur avec précision. En d'autres termes, cela permet de déduire si l'utilisateur est dans un état relaxé ou un état agité par exemple.

Selon un mode de réalisation, les enregistrements des volumes respiratoires sont réalisés à l'aide d'une ceinture abdominale permettant de mesurer une expansion et une contraction thoracique ou abdominale de l'utilisateur et ainsi générer une onde de respiration correspondant aux variations d'un volume respiratoire.

Une telle disposition permet d'obtenir des enregistrements de volumes respiratoires plus précis.

Selon un mode de réalisation, la ceinture abdominale est située au niveau du sternum. Une telle disposition permet d'obtenir des enregistrements de volumes respiratoires plus précis.

Selon un mode de réalisation, chaque première étape de stimulation visuelle comprend une première sous étape de repos réalisée après la stimulation visuelle, la première sous étape de repos consistant à ne plus réaliser de stimulation pendant un premier temps prédéfini.

Selon un mode de réalisation, le premier temps prédéfini est calculé sur la base de la fréquence respiratoire de l'utilisateur et correspond à un premier nombre de cycles prédéfinis. Une telle disposition permet d'obtenir un procédé de caractérisation d'une stimulation olfactive précis, et cela indépendamment de la fréquence respiratoire de l'utilisateur.

Selon un mode de réalisation, la deuxième étape d'enregistrement de mesures de référence est réalisée pendant une sous étape de repos. Une telle disposition permet d'obtenir un deuxième enregistrement de référence plus fiable.

Selon un mode de réalisation, le premier temps prédéfini est de quarante secondes. Une telle disposition permet de réaliser une première étape de stimulation visuelle d'une durée de trente secondes et de laisser à l'utilisateur dix secondes pour se calmer et/ou exprimer un quelconque inconfort.

Selon un mode de réalisation, chaque deuxième étape de stimulation olfactive comprend une deuxième sous étape de repos réalisée après la stimulation olfactive, la deuxième sous étape de repos consistant à ne plus réaliser de stimulation pendant un deuxième temps prédéfini.

Une telle disposition permet de laisser à l'utilisateur le deuxième temps prédéfini pour se calmer et/ou exprimer un quelconque inconfort.

Selon un mode de réalisation, le deuxième temps prédéfini est de trente secondes. Une telle disposition permet d'obtenir une deuxième étape d'enregistrement de valeurs de variables physiologiques plus longue, ce qui permet la réalisation de certains calculs utilisant les valeurs de variables physiologiques avec une plus grande précision. Il est, par exemple, possible de calculer la variabilité de la fréquence cardiaque de l'utilisateur avec plus de précision.

Selon un mode de réalisation, les enregistrements physiologiques sont découpés et filtrés.

Selon un mode de réalisation, les enregistrements physiologiques sont découpés par phase, chaque phase correspondant à une ou plusieurs étapes du procédé. Selon un mode de réalisation préféré, il existe une première phase correspondant à la pluralité de premières étapes de stimulation visuelle ainsi qu'à la pluralité de premières étapes d'enregistrement de valeurs de variables physiologiques, et une deuxième phase correspondant à la deuxième étape de stimulation olfactive ainsi qu'à la deuxième étape d'enregistrement de valeurs de variables physiologiques.

Selon un mode de réalisation, les enregistrements physiologiques peuvent être filtrés de toutes sortes telles que par des fonctions de « smoothing » ou de correction d'artéfacts par exemple. Une telle disposition permet d'obtenir des enregistrements physiologiques de meilleure qualité.

Selon un mode de réalisation, une variabilité de la fréquence cardiaque est calculée dans le domaine fréquentiel et temporel.

Selon un mode de réalisation, une variabilité de la fréquence cardiaque est calculée dans le domaine fréquentiel à l'aide d'une transformée de Fourier à partir du temps entre les battements cardiaques. Un calcul de puissance dans le domaine fréquentiel est par la suite effectué. Une telle disposition permet de mettre en avant certaines caractéristiques des enregistrements physiologiques et ainsi obtenir une caractérisation d'une stimulation olfactive plus précise.

Selon un mode de réalisation, les conductances cutanées phasiques et tonique sont calculées et représentent la mesure de la réaction électrodermale.

Une telle disposition permet, sur la base de la mesure de la réaction électrodermale, de déduire l'activité du système nerveux sympathique.

Au sens de la présente invention, le terme conductance cutanée signifie réponse cutanée galvanique.

Selon un mode de réalisation, une étape d'analyse statistique de référence est réalisée en comparant des moyennes d'enregistrements physiologiques issus de différentes étapes de d'enregistrement de valeurs de variables physiologiques.

Une telle disposition permet de mettre en évidence les différentes perceptions entre les stimulations et ainsi regrouper les stimulations caractérisées.

Selon un mode de réalisation, le procédé de caractérisation d'une stimulation olfactive comprend également un étape d'analyse statistique réalisée entre les stimulations. L'étape d'analyse statistique est effectuée en normalisant les variations entre avant et après la stimulation. La normalisation peut être réalisée de toutes sortes telle que selon la formule suivante :

$$Vn = \frac{X - Y}{Y}$$

Avec :

- Vn : variation normalisée de l'une quelconque des variables physiologiques ou l'une quelconque de ses dérivées ;
- X : une valeur de l'une quelconque des variables physiologiques ou l'une quelconque de ses dérivées après une stimulation ; et
- Y : une valeur de l'une quelconque des variables physiologiques ou l'une quelconque de ses dérivées avant une stimulation.

Une étude statistique est réalisée pour chacune des variables physiologiques et chacune de ses dérivées afin de comparer les différentes stimulations.

Selon un mode de réalisation l'étude statistique comprend l'utilisation d'une méthode statistique telle que l'analyse de la variance.

Selon un mode de réalisation, l'étude statistique comprend l'utilisation d'un test statistique tel que le test de Student.

Les différents aspects définis ci-dessus non incompatibles peuvent être combinés.

**Brève description des figures**

L'invention sera encore mieux comprise à l'aide de la description détaillée qui est exposée ci-dessous en regard du dessin annexé dans lequel :

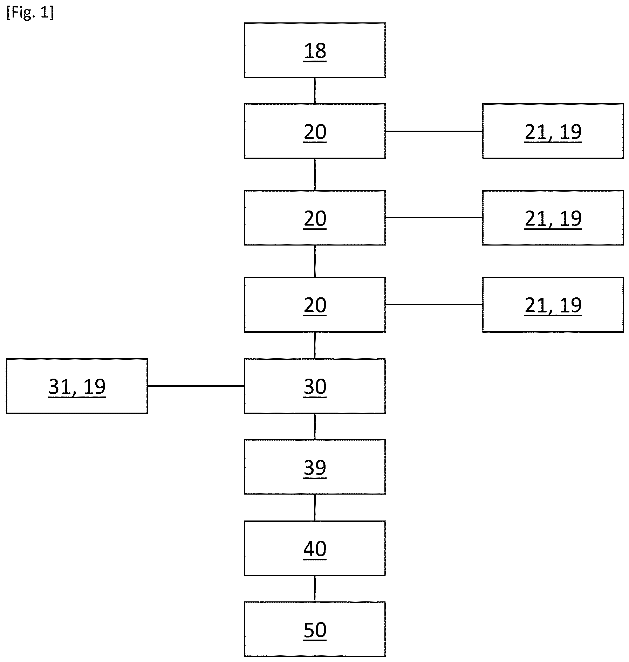

[Fig. 1] représente un diagramme séquentiel d'un procédé de de caractérisation d'une simulation olfactive conformément à la présente invention.

**Description en référence aux figures**

La figure 1 représente les étapes du procédé de caractérisation d'une stimulation olfactive. Ledit procédé comprenant une pluralité de premières étapes de stimulation visuelle 20, chaque première étape de stimulation visuelle 20 consistant à réaliser une stimulation visuelle en montrant à un utilisateur une vidéo. Une telle disposition permet une meilleure stimulation visuelle. Selon un mode de réalisation préféré, la vidéo est une vidéo en réalité virtuelle, telle disposition permet une meilleure immersion et donc une meilleure stimulation visuelle.

Chaque stimulation visuelle est associée à au moins un thème identifié. Le thème identifié peut-être de toutes sortes tel qu'un type d'émotion ou encore un genre cinématographique par exemple. Le thème identifié est un thème identifié par l'utilisateur. En d'autres termes, c'est l'utilisateur qui choisit quel est le thème identifié associé à chaque stimulation visuelle. Et est donc propre à chaque utilisateur. Une telle disposition permet une bonne caractérisation d'une stimulation olfactive.

Chaque première étape de stimulation visuelle 20 comprend une première sous étape de repos réalisée après la stimulation visuelle, la première sous étape de repos consistant à ne plus réaliser de stimulation pendant un premier temps prédéfini. Le premier temps prédéfini est calculé sur la base de la fréquence respiratoire de l'utilisateur et correspond à un premier nombre de cycles prédéfinis. Une telle disposition permet d'obtenir un procédé de caractérisation d'une stimulation olfactive précis, et cela indépendamment de la fréquence respiratoire de l'utilisateur. Avantageusement, le premier temps prédéfini est de quarante secondes. Une telle disposition permet de réaliser une première étape de stimulation visuelle 20 d'une durée de trente secondes et de laisser à l'utilisateur dix secondes pour s'habituer à la réalité virtuelle.

Le procédé comprend une pluralité de premières étapes d'enregistrement de valeurs de variables physiologiques 21, chaque première étape d'enregistrement étant réalisée en continu pendant une première étape de stimulation visuelle 20 parmi la pluralité de premières de stimulation visuelle, de façon à obtenir un premier enregistrement physiologique, la pluralité d'étapes d'enregistrement de valeurs de variables physiologiques résultant ainsi en l'obtention d'une pluralité de premiers enregistrements physiologiques, chaque premier enregistrement physiologique étant associé à un thème identifié correspondant au thème identifié associé à la première étape de stimulation visuelle 20.

Chaque stimulation visuelle a une durée de trente secondes. Une telle disposition permet de réaliser les étapes d'enregistrement de valeurs de variables physiologiques avec une meilleure précision. Cela permet par exemple de réaliser le calcul de la variabilité de la fréquence cardiaque sur des hautes fréquences ainsi que sur les basses fréquences. Au sens de la présente invention, les basses fréquences sont comprises entre 0,04 Hz et 0,15Hz inclus. Au sens de la présente invention, les hautes fréquences sont supérieures à 0,15Hz et inférieures à 0,4Hz inclus.

Le procédé comprend également une deuxième étape de stimulation olfactive 30, la deuxième étape de stimulation olfactive 30 consistant à réaliser une stimulation olfactive. La deuxième étape de stimulation olfactive consiste à approcher un élément, qui produit une sensation sur l'odorat, à quatre centimètres du nez de l'utilisateur. Une telle disposition permet une bonne stimulation olfactive. Ledit élément est contenu dans un flacon ou sur une mouillette. Au sens de la présente invention, une mouillette peut être de toutes sortes, afin que ladite mouillette puisse être imbibée d'un liquide, tel qu'un papier ou une éponge par exemple. Lors de la deuxième étape de stimulation olfactive 30, le sens de la vue est restreint. Une telle disposition permet de limiter les perturbations telles que des stimulations visuelles naturelles et ainsi obtenir une meilleure stimulation olfactive.

Une deuxième étape d'enregistrement de valeurs de variables physiologiques 31 est ensuite réalisée. La deuxième étape d'enregistrement de valeurs de variables physiologiques 31 est réalisée en continu pendant et postérieurement à la deuxième étape de stimulation olfactive 30 résultant ainsi en l'obtention d'un deuxième enregistrement physiologique. La deuxième étape d'enregistrement est réalisée en continu pendant et postérieurement à la deuxième étape de stimulation olfactive 30, de façon à obtenir un deuxième enregistrement physiologique, cela permet de comparer une première partie du deuxième enregistrement physiologique enregistrée pendant la deuxième étape de stimulation olfactive 30 avec une deuxième partie du deuxième enregistrement physiologique afin de caractériser avec plus de précision ladite stimulation olfactive.

Chaque deuxième étape de stimulation olfactive 30 comprend une deuxième sous étape de repos réalisée après la stimulation olfactive, la deuxième sous étape de repos consistant à ne plus réaliser de stimulation pendant un deuxième temps prédéfini. Une telle disposition permet d'obtenir une deuxième étape d'enregistrement de valeurs de variables physiologiques 31 plus longue, ce qui permet la réalisation de certains calculs utilisant les valeurs de variables physiologiques avec une plus grande précision. Il est, par exemple, possible de calculer la variabilité de la fréquence cardiaque de l'utilisateur avec plus de précision. Selon un mode de réalisation, le deuxième temps prédéfini est de trente secondes. Une telle disposition permet d'obtenir une deuxième étape d'enregistrement de valeurs de variables physiologiques 31 plus longue, ce qui permet la réalisation de certains calculs utilisant les valeurs de variables physiologiques avec une plus grande précision. Il est, par exemple, possible de calculer la variabilité de la fréquence cardiaque de l'utilisateur avec plus de précision.

Les variables physiologiques peuvent être de toutes sortes telles que :

- la fréquence cardiaque dans les hautes et les basses fréquences afin de déterminer l'équilibre entre le système nerveux sympathique et parasympathique ;
- la réponse cutanée galvanique, afin de révéler un état d'éveil ou un état de stress par exemple ; et
- le rythme respiratoire afin de révéler un état physiologique et de conforter les états déduits par les autres variables physiologiques ou dérivés.

Les enregistrements physiologiques comprennent des enregistrements réalisés à l'aide d'un photopléthysmogramme placé une phalange distale d'un majeur et/ou sur une phalange distale d'un pouce. Une telle disposition permet d'obtenir une mesure précise lorsque la phalange distale du majeur est trop petite.

Les enregistrements physiologiques comprennent des enregistrements réalisés à l'aide de deux électrodes de conductance électrodermale sur les phalanges médiales de l'index et de l'annulaire. Une telle disposition permet d'obtenir une mesure précise.

Les enregistrements physiologiques comprennent des enregistrements réalisés à l'aide d'un capteur de température cutanée sur la phalange distale de l'auriculaire. Une telle disposition permet d'obtenir une mesure précise. Un calcul de la moyenne de la température cutanée pour chacune des deux parties de chaque enregistrement physiologique est également effectué.

Les enregistrements physiologiques comprennent des enregistrements des volumes respiratoires. Une telle disposition permet de déduire le rythme respiratoire. 1. Les enregistrements des volumes respiratoires comprennent le rythme respiratoire ainsi que les volumes d'inspiration et d'expiration. Une telle disposition permet de déduire l'état physiologique de l'utilisateur avec précision. En d'autres termes, cela permet de déduire si l'utilisateur est dans un état relaxé ou un état agité par exemple. Les enregistrements des volumes respiratoires sont réalisés à l'aide d'une ceinture abdominale permettant de mesurer une expansion et une contraction thoracique ou abdominale et ainsi générer une onde de respiration correspondant aux variations d'un volume respiratoire. Une telle disposition permet d'obtenir des enregistrements de volumes respiratoires plus précis. Préférablement, la ceinture abdominale est située au niveau du sternum de l'utilisateur, et plus précisément de sorte à ce qu'un boitier de mesure de la ceinture abdominale soit situé au niveau du sternum de l'utilisateur. Une telle disposition permet d'obtenir des enregistrements de volumes respiratoires plus précis.

Les enregistrements physiologiques sont ensuite découpés et filtrés par phase, chaque phase correspondant à une ou plusieurs étapes du procédé. De manière avantageuse, il existe une première phase correspondant à la pluralité de premières étapes de stimulation visuelle 20 ainsi qu'à la pluralité de premières étapes d'enregistrement de valeurs de variables physiologiques 21, et une deuxième phase correspondant à la deuxième étape de stimulation olfactive 30 ainsi qu'à la deuxième étape d'enregistrement de valeurs de variables physiologiques 31. Les enregistrements physiologiques sont filtrés de toutes sortes telles que par des fonctions de « smoothing » ou de correction d'artéfacts par exemple. Une telle disposition permet d'obtenir des enregistrements physiologiques de meilleure qualité.

Une variabilité de la fréquence cardiaque est calculée dans le domaine fréquentiel et temporel à l'aide d'une transformée de Fourier à partir du temps entre les battements cardiaques. Un calcul de puissance dans le domaine fréquentiel est par la suite effectué. Une telle disposition permet de mettre en avant certaines caractéristiques des enregistrements physiologiques et ainsi obtenir une caractérisation d'une stimulation olfactive plus précise.

Les conductances cutanées phasiques et tonique sont calculées et représentent la mesure de la réaction électrodermale. Une telle disposition permet, sur la base de la mesure de la réaction électrodermale, de déduire l'activité du système nerveux sympathique. Au sens de la présente invention, le terme conductance cutanée signifie réponse cutanée galvanique.

Une étape d'analyse statistique de référence 39 est réalisée en comparant des moyennes d'enregistrements physiologiques issus de différentes étapes de d'enregistrement de valeurs de variables physiologiques. Une telle disposition permet de mettre en évidence les différentes perceptions entre les stimulations et ainsi regrouper les stimulations caractérisées.

Le procédé de caractérisation d'une stimulation olfactive comprend également un étape d'analyse statistique non représentée et réalisée entre les stimulations. L'étape d'analyse statistique est effectuée en normalisant les variations entre avant et après la stimulation. La normalisation peut être réalisée de toutes sortes telle que selon la formule suivante :

$$ Vn = \frac{X - Y}{Y} $$

Avec :

Vn : variation normalisée de l'une quelconque des variables physiologiques ou l'une quelconque de ses dérivées ;
X : une valeur de l'une quelconque des variables physiologiques ou l'une quelconque de ses dérivées après une stimulation ; et
Y : une valeur de l'une quelconque des variables physiologiques ou l'une quelconque de ses dérivées avant une stimulation.

Une étude statistique est réalisée pour chacune des variables physiologiques et chacune de ses dérivées afin de comparer les différentes stimulations. L'étude statistique comprend l'utilisation d'une méthode statistique telle que l'analyse de la variance ainsi que l'utilisation d'un test statistique tel que le test de Student par exemple.

Le procédé comprend également une étape de comparaison 40, l'étape de comparaison 40 consistant à comparer les premiers enregistrements physiologiques avec le second enregistrement physiologique, de façon à isoler au moins un premier enregistrement physiologique, ledit au moins un premier enregistrement physiologique isolé étant proche du deuxième enregistrement physiologique.

Enfin, le procédé comprend une étape de détermination 50, l'étape de détermination 50 consistant à déterminer au moins un thème identifié, le thème identifié étant le thème identifié associé à l'au moins une première étape de stimulation visuelle 20 isolé lors de l'étape de comparaison 40. L'étape de détermination 50 est réalisée à l'aide de toute méthode telle que d'une classification hiérarchique ascendante et/ou à l'aide à l'aide d'une analyse en composante principale et/ou à l'aide de la méthode des nuées dynamiques par exemple.

Selon un mode de réalisation préféré, le procédé comprend une première étape d'enregistrement de mesures de référence 18. Ladite première étape d'enregistrement de mesures de référence 18 est réalisée avant l'une quelconque des étapes de stimulation et consiste à mesurer en continu des valeurs de variables physiologiques afin d'obtenir un premier enregistrement de référence. Le procédé comprend également une deuxième étape d'enregistrement de mesures de référence 19, la deuxième étape d'enregistrement de mesures de référence 19 étant réalisée après ou pendant l'une quelconque des étapes de stimulation et consistant à mesurer en continu des valeurs de variables physiologiques afin d'obtenir un deuxième enregistrement de référence. La deuxième étape d'enregistrement de mesures de référence 19 est réalisée pendant une sous étape de repos. Une telle disposition permet d'obtenir un deuxième enregistrement de référence plus fiable.

## Revendications

1. Procédé de caractérisation d'une stimulation olfactive, le procédé comprenant :

   • une pluralité de premières étapes de stimulation visuelle (20), chaque première étape de stimulation visuelle (20) consistant à réaliser une stimulation visuelle, chaque stimulation visuelle étant associée à au moins un thème identifié ;

• une pluralité de premières étapes d'enregistrement de valeurs de variables physiologiques (21), chaque première étape d'enregistrement étant réalisée en continu pendant une première étape de stimulation visuelle (20) parmi la pluralité de premières de stimulation visuelle (20), de façon à obtenir un premier enregistrement physiologique, la pluralité d'étapes d'enregistrement de valeurs de variables physiologiques (21) résultant ainsi en l'obtention d'une pluralité de premiers enregistrements physiologiques, chaque premier enregistrement physiologique étant associé à un thème identifié correspondant au thème identifié associé à la première étape de stimulation visuelle ;

• une deuxième étape de stimulation olfactive (30), la deuxième étape de stimulation olfactive (30) consistant à réaliser une stimulation olfactive ;

• une deuxième étape d'enregistrement de valeurs de variables physiologiques (31), la deuxième étape d'enregistrement de valeurs de variables physiologiques (31) étant réalisée en continu pendant et postérieurement à la deuxième étape de stimulation olfactive (30) résultant ainsi en l'obtention d'un deuxième enregistrement physiologique ; et **caractérisé par**

• une étape de comparaison (40), l'étape de comparaison (40) consistant à comparer les premiers enregistrements physiologiques avec le second enregistrement physiologique, de façon à isoler au moins un premier enregistrement physiologique, ledit au moins un premier enregistrement physiologique isolé étant proche du deuxième enregistrement physiologique ; et

• une étape de détermination (50), l'étape de détermination (50) consistant à déterminer au moins un thème identifié, le thème identifié étant le thème identifié associé à l'au moins une première étape de stimulation visuelle (20) isolé lors de l'étape de comparaison (40).

2. Procédé de caractérisation d'une stimulation olfactive selon la revendication 1, le procédé comprenant une première étape d'enregistrement de mesures de référence (18), la première étape d'enregistrement de mesures de référence (18) étant réalisée avant l'une quelconque des étapes de stimulation et consistant à mesurer en continu des valeurs de variables physiologiques afin d'obtenir un premier enregistrement de référence.

3. Procédé de caractérisation d'une stimulation olfactive selon la revendication 2, le procédé comprenant une deuxième étape d'enregistrement de mesures de référence (19), la deuxième étape d'enregistrement de mesures de référence (19) étant réalisée après ou pendant l'une quelconque des étapes de stimulation et consistant à mesurer en continu des valeurs de variables physiologiques afin d'obtenir un deuxième enregistrement de référence.

4. Procédé de caractérisation d'une stimulation olfactive selon l'une quelconque des revendications 1 à 3, dans lequel les enregistrements physiologiques comprennent des enregistrements réalisés à l'aide d'un photopléthysmogramme placé une phalange distale d'un majeur.

5. Procédé de caractérisation d'une stimulation olfactive selon l'une quelconque des revendications 1 à 4, dans lequel les enregistrements physiologiques comprennent des enregistrements réalisés à l'aide de deux électrodes de conductance électrodermale sur les phalanges médiales de l'index et de l'annulaire.

6. Procédé de caractérisation d'une stimulation olfactive selon l'une quelconque des revendications 1 à 5, dans lequel les enregistrements physiologiques comprennent des enregistrements réalisés à l'aide d'un capteur de température cutanée sur la phalange distale de l'auriculaire.

7. Procédé de caractérisation d'une stimulation olfactive selon l'une quelconque des revendications 1 à 6, dans lequel les enregistrements physiologiques comprennent des enregistrements des volumes respiratoires.

8. Procédé de caractérisation d'une stimulation olfactive selon la revendication 7, dans lequel les enregistrements des volumes respiratoires comprennent le rythme respiratoire ainsi que les volumes d'inspiration et d'expiration.

9. Procédé de caractérisation d'une stimulation olfactive selon l'une quelconque des revendications 7 ou 8, dans lequel les enregistrements des volumes respiratoires sont réalisés à l'aide d'une ceinture abdominale permettant de mesurer une expansion et une contraction thoracique ou abdominale et ainsi générer une onde de respiration correspondant aux variations d'un volume respiratoire.

10. Procédé de caractérisation d'une stimulation olfactive selon l'une quelconque des revendications 1 à 9, dans lequel chaque première étape de stimulation visuelle (20) comprend une première sous étape de repos réalisée après la stimulation visuelle, la première sous étape de repos consistant à ne plus réaliser de stimulation pendant un premier temps prédéfini.

**11.** Procédé de caractérisation d'une stimulation olfactive selon l'une quelconque des revendications 1 à 10, dans lequel chaque deuxième étape de stimulation olfactive (30) comprend une deuxième sous étape de repos réalisée après la stimulation olfactive, la deuxième sous étape de repos consistant à ne plus réaliser de stimulation pendant un deuxième temps prédéfini.

**12.** Procédé de caractérisation d'une stimulation olfactive selon l'une quelconque des revendications 1 à 11, dans lequel les enregistrements physiologiques sont découpés et filtrés.

**13.** Procédé de caractérisation d'une stimulation olfactive selon l'une quelconque des revendications 4 à 12 selon la revendication 4, dans lequel une variabilité de la fréquence cardiaque est calculée dans le domaine fréquentiel et temporel.

**14.** Procédé de caractérisation d'une stimulation olfactive selon l'une quelconque des revendications 1 à 13, dans lequel les conductances cutanées phasiques et tonique sont calculées et représentent la mesure de la réaction électrodermale.

**15.** Procédé de caractérisation d'une stimulation olfactive selon l'une quelconque des revendications 1 à 14, dans lequel une étape d'analyse statistique de référence (39) est réalisée en comparant des moyennes d'enregistrements physiologiques issus de différentes étapes de d'enregistrement de valeurs de variables physiologiques.

**Patentansprüche**

**1.** Verfahren zum Charakterisieren einer olfaktorischen Stimulation, wobei das Verfahren Folgendes umfasst:

• eine Vielzahl von ersten Schritten der visuellen Stimulation (20), wobei jeder erste Schritt der visuellen Stimulation (20) darin besteht, eine visuelle Stimulation durchzuführen, wobei die visuelle Stimulation mit mindestens einem identifizierten Thema verknüpft ist;
• eine Vielzahl von ersten Schritten des Aufzeichnens von Werten physiologischer Variablen (21), wobei jeder erste Schritt des Aufzeichnens kontinuierlich während eines ersten Schritts der visuellen Stimulation (20) unter der Vielzahl von ersten Schritten der visuellen Stimulation (20) durchgeführt wird, um eine erste physiologische Aufzeichnung zu erhalten, wobei die Vielzahl von Schritten zum Aufzeichnen von Werten physiologischer Variablen (21) somit dazu führt, dass die Vielzahl von ersten Schritten des physiologischen Aufzeichnens erhalten werden, wobei jede erste physiologische Aufzeichnung mit einem identifizierten Thema verknüpft ist, das dem identifizierten Thema entspricht, das mit dem ersten Schritt der visuellen Stimulation verknüpft ist;
• einen zweiten Schritt der olfaktorischen Stimulation (30), wobei der zweite Schritt der olfaktorischen Stimulation (30) darin besteht, eine olfaktorische Stimulation durchzuführen;
• einen zweiten Schritt des Aufzeichnens von Werten physiologischer Variablen (31), wobei der zweite Schritt des Aufzeichnens von Werten physiologischer Variablen (31) kontinuierlich während und nach dem zweiten Schritt der olfaktorischen Stimulation (30) durchgeführt wird, was dazu führt, dass eine zweite physiologische Aufzeichnung erhalten wird; und **gekennzeichnet durch**
• einen Schritt des Vergleichens (40), wobei der Schritt des Vergleichens (40) darin besteht, die ersten physiologischen Aufzeichnungen mit der zweiten physiologischen Aufzeichnung zu vergleichen, um mindestens eine erste physiologische Aufzeichnung zu isolieren, wobei die mindestens eine erste, isolierte physiologische Aufzeichnung nahe bei der zweiten physiologischen Aufzeichnung liegt; und
• einen Schritt des Bestimmens (50), wobei der Schritt des Bestimmens (50) darin besteht, mindestens ein identifiziertes Thema zu bestimmen, wobei das identifizierte Thema das identifizierte Thema ist, das mit dem mindestens einen ersten Schritt der visuellen Stimulation (20) verknüpft ist, der während des Schritts des Vergleichens (40) isoliert wird.

**2.** Verfahren zum Charakterisieren einer olfaktorischen Stimulation nach Anspruch 1, wobei das Verfahren einen ersten Schritt des Aufzeichnens von Referenzmessungen (18) umfasst, wobei der erste Schritt des Aufzeichnens von Referenzmessungen (18) vor einem der Schritt der Stimulation durchgeführt wird und darin besteht, kontinuierlich Werte physiologischer Variablen zu messen, um eine erste Referenzaufzeichnung zu erhalten.

**3.** Verfahren zum Charakterisieren einer olfaktorischen Stimulation nach Anspruch 2, wobei das Verfahren einen zweiten Schritt des Aufzeichnens von Referenzmessungen (19) umfasst, wobei der zweite Schritt des Aufzeichnens von Referenzmessungen (19) nach oder während eines der Schritte der Stimulation durchgeführt wird und darin besteht, kontinuierlich Werte physiologischer Variablen zu messen, um eine zweite Referenzaufzeichnung zu erhalten.

4. Verfahren zum Charakterisieren einer olfaktorischen Stimulation nach einem der Ansprüche 1 bis 3, wobei die physiologischen Aufzeichnungen Aufzeichnungen umfassen, die unter Verwendung eines Photoplethysmogramms durchgeführt werden, das auf einem distalen Fingerglied eines Mittelfingers platziert wird.

5. Verfahren zum Charakterisieren einer olfaktorischen Stimulation nach einem der Ansprüche 1 bis 4, wobei die physiologischen Aufzeichnungen Aufzeichnungen umfassen, die unter Verwendung von zwei elektrodermalen Leitfähigkeitselektroden an den medialen Fingergliedern des Zeigefingers und des Ringfingers durchgeführt werden.

6. Verfahren zum Charakterisieren einer olfaktorischen Stimulation nach einem der Ansprüche 1 bis 5, wobei die physiologischen Aufzeichnungen Aufzeichnungen umfassen, die unter Verwendung eines Hauttemperatursensors am distalen Fingerglieds des kleinen Fingers durchgeführt werden.

7. Verfahren zum Charakterisieren einer olfaktorischen Stimulation nach einem der Ansprüche 1 bis 6, wobei die physiologischen Aufzeichnungen Aufzeichnungen von Atemvolumina umfassen.

8. Verfahren zum Charakterisieren einer olfaktorischen Stimulation nach Anspruch 7, wobei die Aufzeichnungen der Atemvolumina den Atemrhythmus sowie die Inspirations- und Exspirationsvolumina umfassen.

9. Verfahren zum Charakterisieren einer olfaktorischen Stimulation nach einem der Ansprüche 7 oder 8, wobei die Aufzeichnungen der Atemvolumina unter Verwendung eines Bauchgurts durchgeführt werden, der die Messung der Ausdehnung und Kontraktion des Brust- oder Bauchraums ermöglicht und somit eine Atemwelle erzeugt, die den Veränderungen eines Atemvolumens entspricht.

10. Verfahren zum Charakterisieren einer olfaktorischen Stimulation nach einem der Ansprüche 1 bis 9, wobei jeder erste Schritt der visuellen Stimulation (20) einen ersten Ruhe-Unterschritt umfasst, der nach der visuellen Stimulation durchgeführt wird, wobei der erste Ruhe-Unterschritt darin besteht, keine Stimulation mehr während einer ersten vordefinierten Zeitdauer durchzuführen.

11. Verfahren zum Charakterisieren einer olfaktorischen Stimulation nach einem der Ansprüche 1 bis 10, wobei der zweite Schritt der olfaktorischen Stimulation (30) einen zweiten Ruhe-Unterschritt umfasst, der nach der olfaktorischen Stimulation durchgeführt wird, wobei der zweite Ruhe-Unterschritt darin besteht, keine Stimulation mehr während einer zweiten vordefinierten Zeitdauer durchzuführen.

12. Verfahren zum Charakterisieren einer olfaktorischen Stimulation nach einem der Ansprüche 1 bis 11, wobei die physiologischen Aufzeichnungen aufgeteilt und gefiltert werden.

13. Verfahren zum Charakterisieren einer olfaktorischen Stimulation nach einem der Ansprüche 4 bis 12 nach Anspruch 4, wobei eine Variabilität der Herzfrequenz im Frequenz- und Zeitbereich berechnet wird.

14. Verfahren zum Charakterisieren einer olfaktorischen Stimulation nach einem der Ansprüche 1 bis 13, wobei die phasischen und tonischen Hautleitwerte berechnet werden und die Messung der elektrodermalen Reaktion darstellen.

15. Verfahren zum Charakterisieren einer olfaktorischen Stimulation nach einem der Ansprüche 1 bis 14, wobei ein Schritt der statistischen Referenzanalyse (39) durch Vergleichen von Durchschnittswerten physiologischer Aufzeichnungen, die aus verschiedenen Schritten der Aufzeichnung von Werten physiologischer Variablen stammen, durchgeführt wird.

## Claims

1. A method for characterizing an olfactory stimulation, the method comprising:

   • a plurality of first visual stimulation steps (20), each first visual stimulation step (20) consisting in carrying out a visual stimulation, each visual stimulation being associated with at least one identified theme;
   • a plurality of first steps of recording values of physiological variables (21), each first recording step being carried out continuously during a first visual stimulation step (20) among the plurality of first visual stimulation steps (20), so as to obtain a first physiological recording, the plurality of steps of recording values of physiological variables (21) thus resulting in obtaining a plurality of first physiological recordings, each first physiological recording being associated with an identified theme corresponding to the identified theme associated with the first visual stimulation step;
   • a second olfactory stimulation step (30), the second olfactory stimulation step (30) consisting in carrying out an olfactory stimulation;
   • a second step of recording values of physiological variables (31), the second step of recording values of physiological variables (31) being carried out continuously during and after the second olfactory stimulation step (30) thus resulting in obtaining a second physiological recording; and **characterized by**

• a comparison step (40), the comparison step (40) consisting in comparing the first physiological recordings with the second physiological recording, so as to isolate at least one first physiological recording, said at least one first isolated physiological recording being close to the second physiological recording; and

• a determination step (50), the determination step (50) consisting in determining at least one identified theme, the identified theme being the identified theme associated with the at least one first visual stimulation step (20) isolated during the comparison step (40).

2. The method for characterizing an olfactory stimulation according to claim 1, the method comprising a first step of recording reference measurements (18), the first step of recording reference measurements (18) being carried out before any one of the stimulation steps and consisting in continuously measuring values of physiological variables in order to obtain a first reference recording.

3. The method for characterizing an olfactory stimulation according to claim 2, the method comprising a second step of recording reference measurements (19), the second step of recording reference measurements (19) being carried out after or during any one of the stimulation steps and consisting in continuously measuring values of physiological variables in order to obtain a second reference recording.

4. The method for characterizing an olfactory stimulation according to any one of claims 1 to 3, wherein the physiological recordings comprise recordings carried out using a photoplethysmogram placed on a distal phalanx of a middle finger.

5. The method for characterizing an olfactory stimulation according to any one of claims 1 to 4, wherein the physiological recordings comprise recordings carried out using two electrodermal conductance electrodes on the medial phalanx of the index and of the ring finger.

6. The method for characterizing an olfactory stimulation according to any one of claims 1 to 5, wherein the physiological recordings comprise recordings carried out using a skin temperature sensor on the distal phalanx of the little finger.

7. The method for characterizing an olfactory stimulation according to any one of claims 1 to 6, wherein the physiological recordings comprise recordings of the respiratory volumes.

8. The method for characterizing an olfactory stimulation according to claim 7, wherein the recordings of the respiratory volumes comprise the respiratory rhythm as well as the volumes of inspiration and expiration.

9. The method for characterizing an olfactory stimulation according to any one of claims 7 or 8, wherein the recordings of the respiratory volumes are carried out using an abdominal belt making it possible to measure a thoracic or abdominal expansion and contraction and thus generate a respiration wave corresponding to the variations in a respiratory volume.

10. The method for characterizing an olfactory stimulation according to any one of claims 1 to 9, wherein each first visual stimulation step (20) comprises a first resting sub-step carried out after the visual stimulation, the first resting sub-step consisting in no longer carrying out stimulation for an initial predefined time.

11. The method for characterizing an olfactory stimulation according to any one of claims 1 to 10, wherein each second olfactory stimulation step (30) comprises a second resting sub-step carried out after the olfactory stimulation, the second resting sub-step consisting in no longer carrying out stimulation for a second predefined time.

12. The method for characterizing an olfactory stimulation according to any one of claims 1 to 11, wherein the physiological recordings are cut and filtered.

13. The method for characterizing an olfactory stimulation according to any one of claims 4 to 12 according to claim 4, wherein a heart rate variability is calculated in the frequency and time domain.

14. The method for characterizing an olfactory stimulation according to any one of claims 1 to 13, wherein the phasic and tonic skin conductances are calculated and represent the measurement of the electrodermal reaction.

15. The method for characterizing an olfactory stimulation according to any one of claims 1 to 14, wherein a reference statistical analysis step (39) is carried out by comparing averages of physiological recordings from different steps of recording values of physiological variables.

[Fig. 1]

```
                            ┌──────────────┐
                            │      18      │
                            └──────┬───────┘
                                   │
       ┌──────────────┐     ┌──────┴───────┐     ┌──────────────┐
                            │      20      ├─────┤    21, 19    │
       └──────────────┘     └──────┬───────┘     └──────────────┘
                                   │
                            ┌──────┴───────┐     ┌──────────────┐
                            │      20      ├─────┤    21, 19    │
                            └──────┬───────┘     └──────────────┘
                                   │
                            ┌──────┴───────┐     ┌──────────────┐
                            │      20      ├─────┤    21, 19    │
                            └──────┬───────┘     └──────────────┘
                                   │
       ┌──────────────┐     ┌──────┴───────┐
       │    31, 19    ├─────┤      30      │
       └──────────────┘     └──────┬───────┘
                                   │
                            ┌──────┴───────┐
                            │      39      │
                            └──────┬───────┘
                                   │
                            ┌──────┴───────┐
                            │      40      │
                            └──────┬───────┘
                                   │
                            ┌──────┴───────┐
                            │      50      │
                            └──────────────┘
```

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20120220857 A1

- WO 2019220428 A1